# EUROPEAN PATENT SPECIFICATION

(11) **EP 1 444 367 B1**
(45) Date of publication and mention of the grant of the patent: **11.02.2009**
(21) Application number: 02802983.3
(22) Date of filing: 11.11.2002
(51) Int. Cl.: C12R 1/19, C12N 15/70, C12N 15/68

(54) **RECA-NEGATIVE AND RHAB-NEGATIVE MICROORGANISM**
RECA-NEGATIV UND RHAB-NEGATIV MIKROORGANISMUS
MICRO-ORGANISME RECA-NEGATIF ET RHAB-NEGATIF

(30) Priority: 15.11.2001 DE 10155928
(43) Date of publication of application: 11.08.2004
(73) Proprietor: Evonik Degussa GmbH, 45128 Essen (DE)
(72) Inventor: MAY, Oliver, 60388 Frankfurt (DE); LIEBETON, Klaus, 64673 Zwingenberg (DE); ECK, Jürgen, 64625 Bensheim (DE)
(86) International application number: PCT/EP2002/011979
(87) International publication number: WO 2003/042412

(56) References cited:
- WO-A-97/49289
- WO-A-98/44134
- US-A- 5 543 307
- WILMS BURKHARD ET AL: "High-cell-density fermentation for production of L-N-carbamoylase using an expression system based on the Escherichia coli rhaBAD promoter." BIOTECHNOLOGY AND BIOENGINEERING, vol. 73, no. 2, 20 April 2001 (2001-04-20), pages 95-103, XP002228440 ISSN: 0006-3592
- WILMS BURKHARD ET AL: "Development of an Escherichia coli whole cell biocatalyst for the production of L-amino acids." JOURNAL OF BIOTECHNOLOGY, vol. 86, no. 1, 2001, pages 19-30, XP002228441 ISSN: 0168-1656 cited in the application

### SEQUENCE LISTING

<110> Degussa AG
<120> recA-negative and recB-negative microorganism
<130> 010351 AM
<140>
   <141>
<160> 5
<170> PatentIn Ver. 2.1
<210> 1
   <211> 25
   <212> DNA
   <213> Artificial sequence
<220>
   <223> Description of the artificial sequence: Primer recA+_01f
<400> 1
   attaatgcat tgcagacctt gtggc 25
<210> 2
   <211> 29
   <212> DNA
   <213> Artificial sequence
<220>
   <223> Description of the artificial sequence: Primer recA+_01r
<400> 2
   attaatgatg cgacccttgt gtatcaaac 29
<210> 3
   <211> 23
   <212> DNA
   <213> Artificial sequence
<220>
   <223> Description of the artificial sequence: Primer recB_01f
<400> 3
   aatgaccagg tgccagaaga gag 23
<210> 4
   <211> 21
   <212> DNA
   <213> Artificial sequence
<220>
   <223> Description of the artificial sequence: Primer recB-01r
<400> 4
   ctcctgatgt cgtcaacacg g 21
<210> 5
   <211> 738
   <212> DNA
   <213> Artificial sequence
<220>
   <223> Description of the artificial sequence: DNA fragment carrying RP4-Ori and R6K-Ori
<400> 5
   gaataaggga cagtgaagaa ggaacacccg ctcgcgggtg ggcctacttc acctatcctg 60
   cccggctgac gccgttggat acaccaagga aagtctacat cttacttctt tgcgtagctg 120
   ttaaatacag cgttgttttg ataaaatcat cattatcatc gataatgctt tcttcaattt 180
   ttttatcctt actctttaat aaagcacttg ctaataactt catacctttt gcaactgtca 240
   aatttggttc atcagggtaa atgcttttaa ggcatactaa caaataatca tggtcttcat 300
   cttcaactct aaactgaatt tttttcatca taactcccaa caagaaccga ctgtaggtca 360
   ccgggcaaac gctgaaaaat aacgtcgaat gacgtcattt tgcggcgttt gccctatcct 420
   gcatcgcagt agaaaatgcc acaactgaaa ttgtgcttca gtatgtacag aaatgcaaaa 480
   tctgagggat ttcgtagctg aaagatcgcc agtcttcgac cgtaaggata ggagttgctg 540
   taagacctgt gcggggcgtt cgcttcgcga acgggtctgg cagggggcac aagcgctgtg 600
   ctgtgatata tgcaaaagaa gccacccacg aacgggaggg cttcggcgaa tcgactatag 660
   tgatctattt acccggctga ttgtcgcctt ctagccctcg cgggcatcat gcaaccagtg 720
   cctgaattta gttatatg                                              738

## Description

The present invention relates to a microorganism which can be used for expression of genes which code for proteins which are to be prepared in a recombinant manner. This host organism has, in particular, a recombination deficiency (recA-) coupled with the absence of an ability to break down rhamnose (rhaB-). The invention furthermore includes a process for the production of the microorganism and preferred uses thereof.

Since the development of recombinant DNA technology, it has been possible to produce proteins inexpensively in recombinant microorganisms (Construction of biologically functional bacterial plasmids in vitro. Cohen, S.N.; Chang, A.C.; Boyer, H.W.; Helling, R.B. (1973), Proc. Natl. Acad. Sci. USA: 70, 3240-3244). Since the start of the 1970s, because of the increasing industrial importance of proteins, expression systems based on various promoters and host organisms have therefore been developed (Expression of heterologous gene products in yeast. Pichuantes, Sergio; Nguyen, Anton Tien; Franzusoff, Alex. Editor(s): Cleland, Jeffrey L.; Craik, Charles S. Protein Eng. (1996), 129-161. Inducible gene expression systems in Lactococcus lactis. Djordjevic G M; Klaenhammer T. R. MOLECULAR BIOTECHNOLOGY (1998 Apr), 9(2), 127-39. Expression systems for industrial Gram-positive bacteria with low guanine and cytosine content. de Vos W M; Kleerebezem M; Kuipers O P. CURRENT OPINION IN BIOTECHNOLOGY (1997 Oct), 8(5), 547-53. Alternative regulation principles for the production of recombinant proteins in Escherichia coli. Sawers G; Jarsch M. APPLIED MICROBIOLOGY AND BIOTECHNOLOGY (1996 Aug), 46(1), 1-9. Strategies for optimizing heterologous protein expression in Escherichia coli. Hannig, Gerhard; Makrides, Savvas C. Trends Biotechnol. (1998), 16(2), 54-60. Strategies for achieving high-level expression of genes in Escherichia coli. Makrides, Savvas C. Microbiol. Rev. (1996), 60(3), 512-538.). A promoter is generally understood as meaning a DNA sequence region from where the transcription of a gene or operon is controlled. A distinction is made between potent and weak promoters. Promoters can be controlled by addition of certain substances to the nutrient medium of the microorganism (Gentechnologie für Einsteiger [Genetic Engineering for Beginners], T.A. Brown, Spektrum, 2nd edition, p. 285). Thus e.g. L-rhamnose is also a substance which can influence a corresponding promoter to the extent of expressing a subsequent gene.

In order thus to switch on the expression of a promoter-gene construct which can be induced by L-rhamnose, L-rhamnose must be fed to the microorganism. This is advantageously effected by addition of L-rhamnose to the nutrient medium on which the organism is growing. However, L-rhamnose moreover also forms a source of carbon to be used for the microorganism and is utilized by the microorganism itself and therefore broken down.

Since L-rhamnose is a relatively expensive inducer, there has been no lack of attempts to minimize the consumption of L-rhamnose for such systems. An L-rhamnose-inducible expression system which achieves frequently better results compared with other systems has recently been published (Ein neues, L-Rhamnose-induzierbares Expressionssytem für Escherichia coli [A New, L-Rhamnose-Inducible Expression System for Escherichia coli]. Stumpp, T.; Wilms, B.; Altenbuchner, Biospektrum (2000), 1, 33-36.). A strain (E. coli BW3110) which has a greatly reduced rhamnose metabolism due to mutation in the rhamnulose kinase gene rhaB has been produced (Development of an Escherichia coli whole cell biocatalyst for the production of L-amino acids. Wilms, B.; Wiese, A.; Syldatk, C.; Mattes, R.; Altenbuchner, J. (2001), J.Biotechnol. 86, 19-30). Disadvantages for the industrial use of this expression system (combination of rhamnose promoter and E. coli BW3110) are already listed in Stumpp et al.:
- The specific activity of the enzyme formed (hydantoinase) in the crude extract remained 15-25% below that of an isogenic E. coli strain which consumes rhamnose.
- Since E. coli BW3110 has a recombination system (recA), an extreme plasmid instability was found: 70% of the cells no longer contained plasmid after fermentation without selection pressure. Although this genetic instability for plasmids can be improved by known stabilization systems, the problem of homologous recombinations within the bacterial chromosome remains.

The object was thus to create a microorganism which on the one hand requires less L-rhamnose for induction of gene expression, but in which on the other hand the genetic instability known from the prior art is at least reduced. In particular, it should be possible to use the microorganism on an industrial scale for the production of rec proteins in an economically favourable manner.

The object is achieved according to the claims. Claims 1 to 4 relate to the microorganism according to the invention. Claims 5 and 6 protect a particular production process for this, while claims 7 to 9 relate to specific uses thereof. Claim 10 relates to individual vectors which are to be used in the synthesis of the microorganism.

By providing a microorganism which has a recombination deficiency (recA-) and a deficiency in the breakdown of rhamnose (rhaB-) which has taken place due to deletion of the rhaB gene, the object described was achieved in an advantageous but nevertheless not readily foreseeable manner.

The microorganism is used merely for multiplying and producing a sufficient amount of the recombinant protein. The processes for carrying out these measures are well-known to the expert (Sambrook et al. 1989, Molecular cloning: A Laboratory Manual, 2nd Edition, Cold Spring Harbor Laboratory Press, Balbas P & Bolivar F. 1990, Design and construction of expression plasmid vectors in E. coli, Methods Enzymology 185, 14-37).

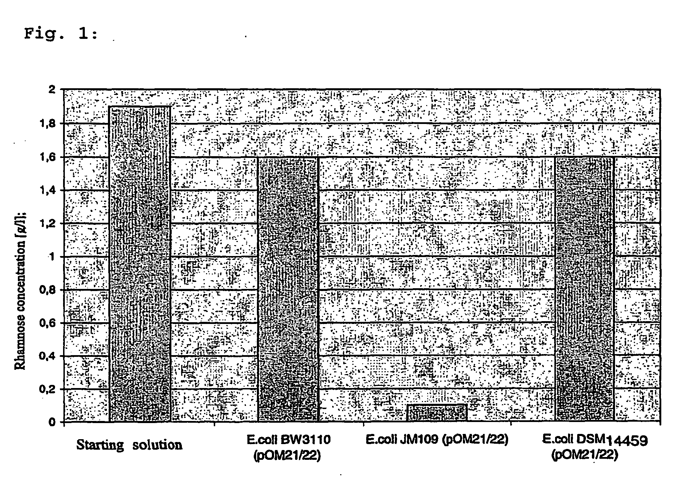

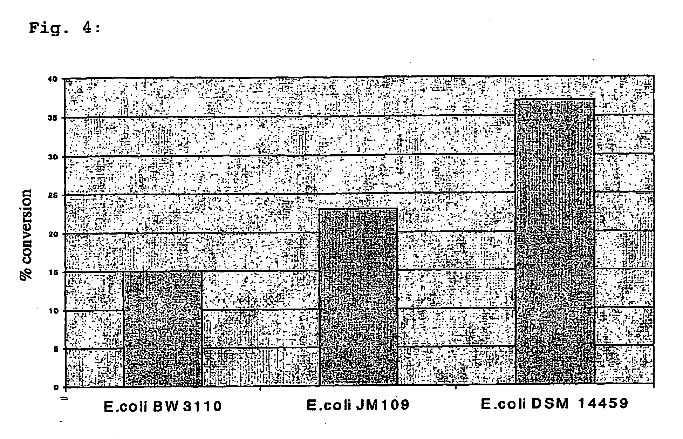

The organism according to the invention can be used in an excellent manner for the production of recombinant proteins (fig. 4), it being possible for the amount of L-rhamnose to be used for the induction of gene expression to be reduced significantly, since the microorganism consumes none of this during growth. Comparison of the strains in fig. 1 in respect of rhamnose breakdown shows that both E. coli BW3110 and the newly produced strain E. coli DSM 14459 scarcely metabolize the inducer L-rhamnose, as a result of which, as already shown for E. coli BW3110, a 90% reduction in the rhamnose consumption is possible (Stummp et al.).

In principle, any organism which is advantageously to be used for the production of rec proteins can be modified according to the invention. Organisms such as e.g. prokaryotes or eukaryotes, such as Pseudomonas, Streptomyces, Arthrobacter, Bacillus, Staphylococcus, Escherichia, Candida, Hansenula and Pichia, can be used for this purpose. An E. coli is preferably to be used. The following are very particularly preferred: E. coli NM 522, BL21, XL1 Blue, JM101, JM109. JM105, RR1, DH5α, TOP 10- or HB101. Of these, those which already have an recA deficiency are extremely preferred.

The microorganism which was deposited under number DSM 14459 at DSMZ GmbH [German Collection of Microorganisms and Cell Cultures GmbH], Mascheroder Weg 1b, D-38124 Braunschweig on 24.08.01 in accordance with the Budapest Treaty is very particularly advantageous. A microorganism according to the invention containing the vectors pOM21 and pOM22 (PCT/US00/08159) is moreover extremely preferred.

As already indicated above, the microorganism according to the invention is preferably suitable for the production of rec proteins, gene constructs which have a rhamnose-inducible promoter being used for the expression. A microorganism according to the invention containing at least one rhamnose-inducible expression system is therefore very particularly preferred. The term expression system in this case is understood as meaning a vector which can be replicated in the microorganism according to the invention in a stable manner and is equipped with a rhamnose-inducible promoter. Such vectors can preferably be derived from pUC, pBR, pSC101 and pACYC derivatives and can be found in a correspondingly modified form, for example, in Studier et al., Methods Enzymol. 1990, 185, 61-69 or the brochures of Roche Biochemicals, Invitrogen, Novagen, Promega, New England Biolabs, Clontech or Gibco BRL. Further preferred vectors can be found in: DNA cloning: a practical approach. volume I-III, edited by D. M. Glover, IRL Press Ltd., Oxford, Washington DC, 1985, 1987; Denhardt, D. T. and Colasanti, J.: A survey of vectors for regulating expression of cloned DNA in E. coli. In: Rodriguez, R.L. and Denhardt, D. T (eds), Vectors, Butterworth, Stoneham, MA, 1987, pp179-204; Gene expression technology. In: Goeddel, D. V. (eds), Methods in Enzymology, volume 185, Academic Press, Inc., San Diego, 1990; Sambrook, J., Fritsch, E.F. and Maniatis, T. 1989. Molecular cloning: a laboratory manual, 2nd ed. Cold Spring Harbor Laboratory Press, Cold Spring Harbor, N.Y.

Vectors with a rhamnose-inducible promoter with which the gene construct containing the nucleic acid to be expressed can be cloned in a very preferred manner into the host organism are: pKK-177-3H (Roche Biochemicals), pBTac (Roche Biochemicals), pKK-233-3 (Amersham Pharmacia Biotech), pLex (Invitrogen) or the vectors of the pET series (Novagen). The vectors pOM21 and pOM22 (PCT/US00/08159) e.g. are very particularly preferred.

The present invention also provides a process for the production of the microorganisms according to the invention, in which the mutagenesis construct for inactivation of the rhamnulokinase gene is transferred conjugatively into the recombination-active microorganism and, after successful integration, the recombination activity is eliminated in a targeted manner.

To produce the desired phenotype, the expert could, *inter alia*, delete the rhaB gene analogously to the production of the strain E. coli BW3110 (Development of an Escherichia coli whole cell biocatalyst for the production of L-amino acids. Wilms, B.; Wiese, A.; Syldatk, C.; Mattes, R.; Altenbuchner, J. (2001), J.Biotechnol. 86, 19-30.) in a targeted manner, a strain with an recA-negative phenotype being resorted to, in contrast to the production of E. coli BW3110. However, the recA-negative phenotype resulted in several problems in the production of the desired strain which were unexpected to the expert:

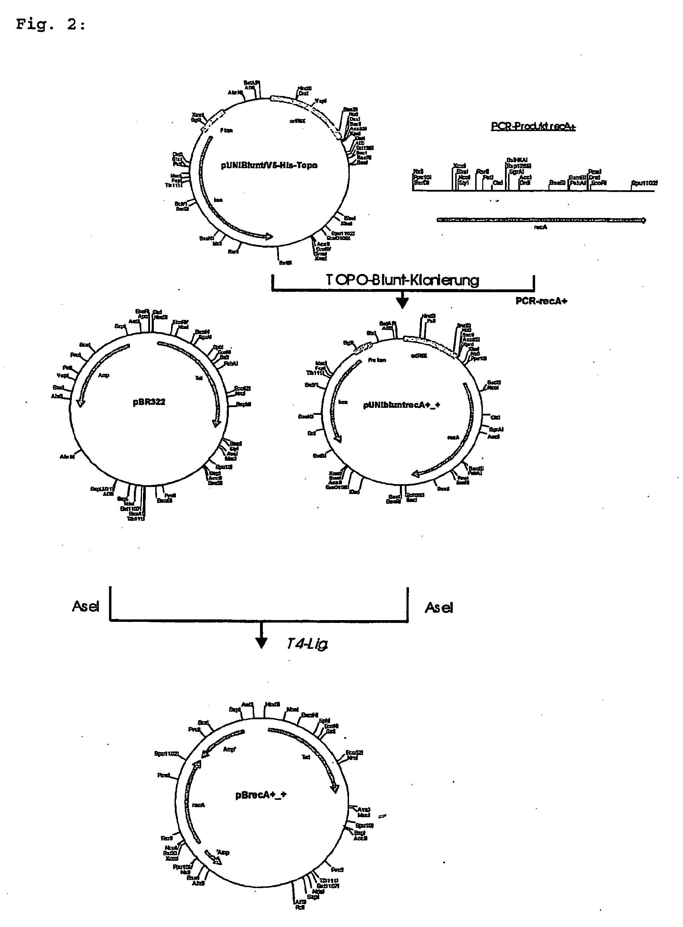

A prerequisite for mutagenesis via homologous recombination is the re-establishment of the recA-positive phenotype in E. coli JM109. For this, the recA gene from the strain E. coli BL21 can be amplified by means of PCR and ligated, for example, with the vector pBR322 and pUniBlunt/V5-His-TOPO (fig. 2). These constructs impart to the strain E. coli JM109 an recA-positive phenotype, as induction of the SOS response after UV irradiation demonstrates (see example 1). A mutagenesis construct (e.g. pMut1, see example 1, fig, 3) for inactivation of the rhamnulokinase gene (rhaB) can be established, for example, by ligation of a part sequence of the rhaB gene, into which a reading frame shift is introduced, in a vector which cannot be replicated in E. coli JM109 (suicide vector). However, it was not possible to prepare an rhaB-negative mutant of the strain JM109 + pBrecA+, which has an recA-positive phenotype due to the plasmid pBrecA+, by this procedure by transformation of the mutagenesis construct.

The construction of an rhaB-negative mutant of E. coli JM109 by a recombinase system (a system which is independent of recA; analogous to One-step inactivation of chromosomal genes in Escherichia coli K - 12 using PCR products. Datsenko, Kirill A.; Wanner, Barry L. Proc. Natl. Acad. Sci. U. S. A. (2000), 97(12), 6640-6645.) also did not lead to the aim.

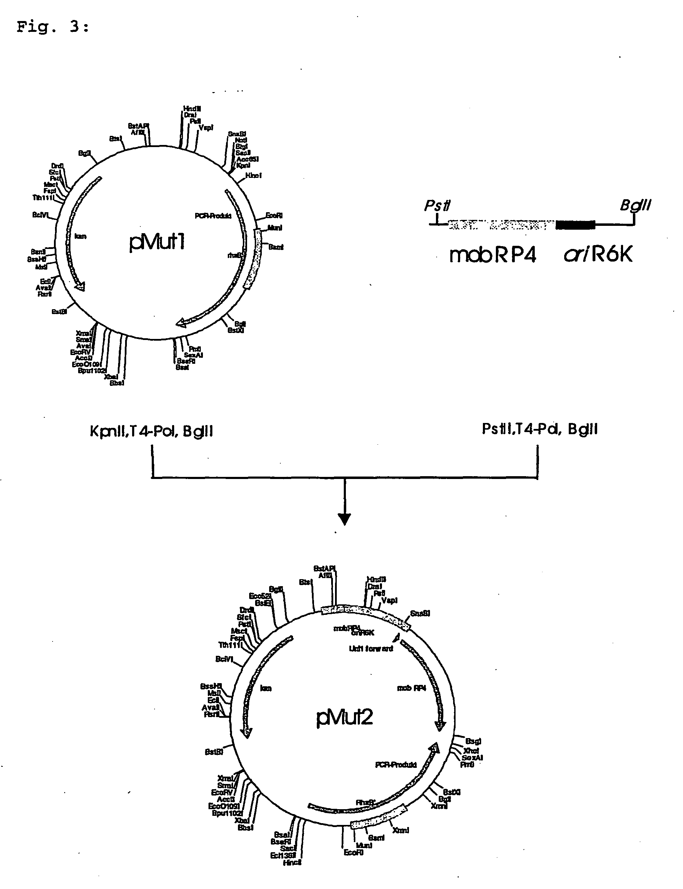

However, the aim was arrived at when, after insertion of the RP4 "origins of replication" (Conditionally replicative and conjugative plasmids carrying lacZα for cloning, mutagenesis, and allele replacement in bacteria. Metcalf, William W.; Jiang, Weihong; Daniels, Larry L.; Kim, Soo-Ki; Haldimann, Andreas; Wanner, Barry L. Plasmid (1996), 35(1), 1-13.) into the vector pMut1, the resulting mutagenesis construct pMut2 (fig. 3) transfers conjugatively (Sambrook et al. 1989, Molecular cloning: A Laboratory Manual, 2nd Edition, Cold Spring Harbor Laboratory Press, Balbas P & Bolivar F. 1990) to E. coli JM109 + pBrecA+ and integrates into the chromosome. As expected, both rhaB-negative and rhaB-positive transconjugants can be identified with pMut2, which manifests itself in the white and red phenotype on the indicator medium (see example 1).

Starting from an rhaB-negative transconjugant (or rhaB-positive transconjugant and subsequent excision of the integrated vector), those clones which, after incubation several times without selection pressure for the antibiotic resistance (tetracycline) imparted by the vector pBrecA+, are tetracycline-sensitive can be concentrated. By induction of the SOS response by means of UV irradiation, the recA phenotype thereof can be investigated and confirmed as recA-negative. Clones in which the phenotype can be summarized as rhaB-negative and recA-negative can be identified in this way.

In a next embodiment, the invention relates to the use of the microorganism according to the invention in a process for the production of rec proteins. In principle, the expert has a free choice of the rec proteins to be produced. The processes are to be found in the general technical literature (see: Sambrook et al. 1989, Molecular cloning: A Laboratory Manual, 2nd Edition, Cold Spring Harbor Laboratory Press, Balbas P & Bolivar F. 1990; Design and construction of expression plasmid vectors in E. coli, Methods Enzymology 185, 14-37; Vectors: A Survey of Molecular Cloning Vectors and Their Uses. R.L. Rodriguez & D.T. Denhardt, eds: 205-225). In respect of the general procedures (PCR, cloning, expression etc.) reference may also be made to the abovementioned literature and that cited there.

The use of the microorganism for the production of enantiomerically concentrated amino acids analogously to a whole cell catalyst, as disclosed in PCT/EP00/08473 and PCT/US00/08159, is particularly preferred. The embodiment in which the microorganism according to the invention has a rec protein with the property of a hydantoinase, a carbamoylase and/or a hydantoin or carbamoyl racemase is therefore extremely preferred. It is thus possible to generate enantiomerically concentrated amino acids from hydantoins which are easy to synthesize. The microorganism used in this way can advantageously contain the vectors pOM21 and pOM22, as realized in E. coli DSM 14459 (pOM21/pOM22) (PCT/US00/08159). E. coli DSM 14459, E. coli JM109 and E. coli BW3110 were each transformed with pOM21 and pOM22 and compared in respect of rhamnose consumption and activity (fig. 1/4). Fig. 4 shows that the recombinant strain DSM 14459 has a significantly higher activity not only compared with the recombinant starting strain E. coli JM109 (pOM21, pOM22) but surprisingly also compared with E. coli BW3110 (pOM21, pOM22). In this connection, it is interesting that it has been reported for E. coli BW3110 that, in the crude extract, the specific activity of an enzyme formed remains 15-25% below that of an isogenic strain which consumes rhamnose (Ein neues, L-Rhamnose-induzierbares Expressionssystem für Escherichia coli [A New, L-Rhamnose-Inducible Expression System for Escherichia coli. Stumpp, T.; Wilms, B.; Altenbuchner, Biospektrum (2000), 1, 33-36.).

Another aspect of the invention is the fact that further mutations in the genome of the microorganism according to the invention can lead to mutants or variants which have further improved properties. The present invention therefore also provides the use of the microorganism according to the invention for the production of mutants or variants of the microorganism. The processes for the introduction are adequately known to the expert (see the lit. cited in this specification).

The organism according to the invention can be transformed with all the vectors possible to the expert for this purpose. The construction of suitable vectors can be found from general technical knowledge. Specific strategies are described e.g. in: One-step inactivation of chromosomal genes in Escherichia coli K - 12 using PCR products. Datsenko, Kirill A.; Wanner, Barry L. Proc. Natl. Acad. Sci. U.S.A. (2000), 97(12), 6640-6645; A genome-based approach for the identification of essential bacterial genes. Arigoni, Fabrizio; Talabot, Francois; Peitsch, Manuel; Edgerton, Michael D.; Meldrum, Eric; Allet, Elisabeth; Fish, Richard; Jamotte, Therese; Curchod, Marie-Laure; Loferer, Hannes. Nat. Biotechnol. (1998), 16(9), 851-856. The following vectors are preferably used for the modification according to the invention of the microorganisms: pBrecA+, pMut1 and pMut2. The invention also provides the production of a combination of an recA-negative and rhaB-negative phenotype by targeted knockout of rhaB by methods known to the expert using recA-positive strains and subsequent inactivation of recA.

For the uses according to the invention, e.g. for the preparation of amino acids, the rec enzymes in question prepared according to the invention can be used in the free form as homogeneously purified compounds. The rec enzyme can furthermore also be employed as a constituent of the intact guest organism or in combination with the broken down cell mass of the host organism, which has been purified to any desired extent. The use of the enzymes in immobilized form is also possible (Bhavender P. Sharma, Lorraine F. Bailey and Ralph A. Messing, "Immobilisierte Biomaterialien - Techniken und Anwendungen [Immobilized Biomaterials - Techniques and Uses]", Angew. Chem. 1982, 94, 836-852). The immobilization is advantageously carried out by lyophilization (Dordick et al., J. Am. Chem. Soc. 1994, 116, 5009-5010; Okahata et al., Tetrahedron Lett. 1997, 38, 1971-1974; Adlercreutz et al., Biocatalysis 1992, 6, 291-305). Lyophilization in the presence of surface-active substances, such as Aerosol OT or polyvinylpyrrolidone or polyethylene glycol (PEG) or Brij 52 (diethylene glycol mono-cetyl ether) is very particularly preferred (Goto et al., Biotechnol. Techniques 1997, 11, 375-378). Use as CLECs is also conceivable (St Clair et al., Angew Chem Int Ed Engl 2000 Jan, 39(2), 380-383).

In the context of the invention, optically concentrated (enantiomerically concentrated, enantiomer-concentrated) compounds is understood as meaning the presence of an optical antipode as a mixture with the other in >50 mol%.

A natural amino acid is one such as is described in Beyer-Walter, Lehrbuch der organischen Chemie [Textbook of Organic Chemistry], S. Hirzel Verlag Stuttgart, 22nd edition, 1991, p. 822 et seq. Furthermore, however, corresponding non-natural α-amino acids, such as are listed e.g. in DE19903268.8, are also referred to.

### Examples:

### 1. Process for the production of an recA- and rhaB- E. coli

### 1.1 Preparation of genomic DNA from E. coli JM109 and E. coli BL21 (DE3)

5 ml LB liquid medium were inoculated with a colony and incubated at 37°C, 180 rpm for 16 hours. The preparation of genomic DNA was carried out by means of a kit (Cat# 6560-200) from Bio 101 (Vista, USA) in accordance with the protocol contained therein.

### 1.2 Amplification of recA from genomic DNA of E. coli BL21 (DE3) and rhaB from genomic DNA of E. coli JM109 by means of PCR

PCR batch:
Approx. 100 ng genomic DNA from 1.1; in each case 20 pmol primer recA+_01f (Seq. ID 1) and primer recA±_01r (Seq. ID 2) for amplification of the recA gene or 20 pmol primer rhab_01f (Seq. ID 3) and primer rhab_01r (Seq. ID 4) for amplification of the rhaB gene; 2.5 U Hot Star Taq polymerase (Qiagen, Germany); other conditions in accordance with the instructions of the Hot Star Taq Polymerase Kit from Qiagen (Germany).

PCR cycles:
1x (15 min 95°C)
30x (30 s 95°C, 30 s 48°C, 60 s 72°C)
1x (7 min 72°C)

The purification of the PCR fragments was carried out by means of the Min Elute PCR Purification Kit (Qiagen, Germany) in accordance with the instructions.

### 1.3 Cloning of the rhaB and recA fragments into pUniBlunt/V5-His-TOPO (construction of pUnirecA+ and pRhaB)

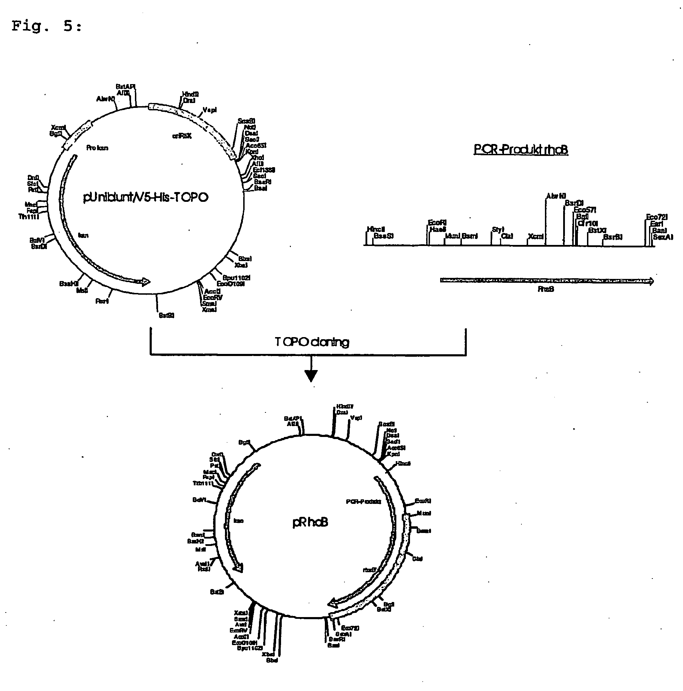

Cloning of the PCR fragments resulting from 1.2 and transformation of the ligation products in E. coli PIR1 were carried out by means of the pUni/V5-His-TOPO cloning kit (can.#; ET004-xx) from Invitrogen (Carlsbad, USA) in accordance with the instructions contained therein. The plasmid resulting from rhaB and pUniBlunt/V5-His-TOPO is called pRhaB (fig. 5). The plasmid resulting from recA and pUniBlunt/V5-His-TOPO is called pUnirecA+.

### 1.4 Construction of pMut1 starting from pRhaB

pRhaB was cleaved with the restriction enzyme ClaI from Roche (Germany) in accordance with the instructions included, The projecting ends of the resulting vector fragment were completed with T4 DNA polymerase from New England Biolabs (Frankfurt, Germany) in accordance with the instructions included.

### 1.5 Construction of pMut2 starting from pMut1

pMut1 was digested with KpnI and BglII and the overhanging ends were completed with the aid of T4 DNA polymerase. The fragment approx. 2.6 kb in size was purified by means of a Qiagen Gel Extraction Kit and ligated with a DNA fragment carrying RP4-Ori and R6K-ori (Seq. ID 5) using a T4 DNA ligase in accordance with the instructions (see above).

### 1.6 Preparation of an recA-positive E. coli JM109

E. coli JM109 was transformed with the plasmid pBrecA+ in accordance with a standard protocol (e.g. described in: Sambrook, J., Fritsch, E.F. and Maniatis, T. 1989. Molecular cloning: a laboratory manual, 2nd ed. Cold Spring Harbor Laboratory Press, Cold Spring Harbor, N.Y.).

Transformants were tested for RecA activity on an LB agar plate by UV irradiation (254 nm, 40 cm distance, 5-20 seconds). Positive (recA-active) clones still showed significant growth at an increasing duration of the irradiation, in contrast to E. coli JM109 wild-type. recA-positive clones are called E. coli JM109 + pBrecA+.

### 1.7 Conjugative transfer of the mutagenesis construct pMut2 and screening for the rhaB-negative phenotype

2 ml of stationary cultures of E. coli S17-1 (λpir), which had been transformed with pMut2 in accordance with the standard protocol (see above), and E. coli JM109 + pBrecA+ were harvested by centrifugation and washed 3x with LB medium. After resuspending the particular cells, these were mixed and incubated for 16 h on LB plates at 37ºC. The cells were then flushed off from the plate and plated out on LB-Kan,Tet plates. After 16-24 hours transconjugants in which the mutagenesis construct had been integrated into the chromosome were visible. Approx 25% of the transconjugants were white when plated on to MacConkey rhamnose agar plates (composition in g/litre: peptone 20.0; NaCl 5.0; bile salt 1.5; rhamnose 10.0; crystal violet 0.001; neutral rot 0.03; agar-agar 15.0) and therefore showed an rhaB-negative phenotype.

### 1.8 Concentration of a tetracycline-sensitive, recA-negative phenotype

The rhaB-negative clones produced in 1.7 have an recA-positive phenotype due to the plasmid pBrecA+. In order now to obtain recA-negative cells, the rhaB-negative clones obtained in 1.7 were cultured over several generations with the addition of tetracycline (12.5 µg/ml) and ampicillin (100 µg/ml) without selection pressure for the tetracycline resistance imparted by the plasmid pBrecA. (Note: Only cells which have lost the plasmid pBrecA+ can survive through this negative selection.) After harvesting of the cells and washing three times with LB medium, the cells were applied to LB agar and incubated for 16 hours at 37 ºC. The loss of pBrecA+ from the washed clones could then be confirmed by tetracycline sensitivity and UV irradiation to detect the recA-negative phenotype analogously to the procedure in 1.6. It was thus possible to produce E. coli clones with an recA-negative and rhaB-negative phenotype, such as, for example, E. coli DSM 14459.

### 2. Determination of the enzyme activity of whole cell biocatalysts (see fig. 4)

Composition of the growth medium:
10 g/l tryptone
5 g/l yeast extract
10 g/l NaCl
100 mg/l ampicillin
50 mg/l chloramphenicol
2 g/l rhamnose
1 mM CoCl₂

Composition of the reaction solution:
100 mM DL-methylthioethylhydantoin (MTEH)
0.1 mM CoCl₂
pH 7.8

Overnight cultures of E. coli DSM 14459 (pOM21, pOM22), E. coli JM109 (pOM21, pOM22) and E. coli BW3110 (pOM21, pOM22) were diluted 1:100 in 20 ml growth medium and incubated at 30 ºC at 250 rpm. The growth was monitored over 24 h. After approx. 24 h an optical density measured at 600 nm (OD600) of 4 was reached and the cells were pelleted by means of centrifugation (5 min, 15,000 g). The particular cell pellets were then suspended in the reaction solution such that an OD600 of 10 resulted and were incubated immediately at 37 ºC, 1,000 rpm for 10 minutes. Thereafter, the reaction was stopped by addition of HPLC mobile phase (see below) and the cells were separated off from the reaction supernatant by means of centrifugation (5 min, 15,000 g). The concentration of methionine formed was determined in the reaction supernatant by means of HPLC (see below) and the percentage conversion (mol/mol) was calculated.

HPLC method:
Column: RP-18 (Waters)
HPLC mobile phase: 985 ml H₂O, 15 ml acetonitrile, 1 ml trifluoroacetic acid
Flow rate: 1 ml/min
Detection: 220 nm

### 3. Determination of the rhamnose consumption (see fig. 1)

Overnight cultures of E. coli DSM 14459 (pOM21, pOM22), E. coli JM109 (pOM21, pOM22) and E. coli BW3110 (pOM21, pOM22) were diluted 1:100 in 20 ml growth medium and incubated at 30ºC at 250 rpm. The growth was monitored over 24 h. After approx. 24 h an optical density measured at 600 nm (OD600) of 4 was reached and the cells were pelleted by means of centrifugation (5 min, 15,000 g). The supernatant was analysed for the rhamnose content by means of HPLC.

HPLC method:
Column: Aminex HPX-87 H (BioRad)
HPLC mobile phase: 5 mM H₂SO₄
Flow rate: 0,6 ml/min
Detection: Refractive index (RI detector)

## Claims

1. Microorganism containing a recombination deficiency (recA-) and a deficiency in the breakdown of rhamnose (rhaB-) which has taken place due to a deletion of the rhaB gene.

2. Microorganism according to claim 1, **characterized in that** it is E. coli.

3. Microorganism according to claim 1 and 2, deposited under number DSM 14459.

4. Microorganism according to one or more of claims 1 to 3, containing at least one rhamnose-inducible Expression system.

5. Process for the production of microorganisms according to claims 1 to 4,
**characterized in that**
the mutagenesis construct for inactivation of the rhamnulokinase gene is transferred conjugatively into the recombination-active microorganism and, after successful integration, the recombination activity is eliminated in a targeted manner.

6. Process according to claim 5, **characterized in that** the conjugation time is adjusted to ≤ 30 min.

7. Use of the microorganism according to one or more of claims 1 to 4 in a process for the production of rec proteins.

8. Use according to claim 7, **characterized in that** the microorganism has a rec protein with the property of a hydantoinase, a carbamoylase and/or a hydantoin or carbamoyl racemase.

9. Use of the microorganism according to one or more of claims 1 to 4 for the production of mutants or variants of the microorganism.

## Patentansprüche

1. Mikroorganismus aufweisend eine Rekombinationsdefizienz (recA-) und eine durch eine Deletion des rhaB-Gens erfolgte Defizienz im Abbau von Rhamnose (rhaB-).

2. Mikroorganismus nach Anspruch 1, **dadurch gekennzeichnet, dass** es sich um E. coli handelt.

3. Mikroorganismus nach Anspruch 1 und 2 hinterlegt unter der Nummer DSM 14459.

4. Mikroorganismus gemäß einem oder mehreren der Ansprüche 1 bis 3, aufweisend mindestens ein rhamnoseinduzierbares Expressionssystem.

5. Verfahren zur Herstellung von Mikroorganismen nach den Ansprüchen 1 bis 4,
**dadurch gekennzeichnet,**
**dass** man das Mutagenesekonstrukt zur Inaktivierung des Rhamnulokinase-Gens konjugativ in den rekombinationsaktiven Mikroorgansimus transferiert und nach erfolgreicher Integration die Rekombinationsaktivität gezielt ausschaltet.

6. Verfahren nach Anspruch 5, **dadurch gekennzeichnet, dass** man die Konjugationszeit auf ≤ 30 min einstellt.

7. Verwendung des Mikroorganismus nach einem oder mehreren der Ansprüche 1 bis 4 in einem Verfahren zur Herstellung von rec-Proteinen.

8. Verwendung nach Anspruch 7, **dadurch gekennzeichnet, dass** der Mikroorganismus ein rec-Protein mit der Eigenschaft einer Hydantoinase, einer Carbamoylase und/oder einer Hydantoin- oder Carbamoylracemase aufweist.

9. Verwendung des Mikroorganismus nach einem oder mehreren der Ansprüche 1 bis 4 zur Herstellung von Mutanten oder Varianten des Mikroorganismus.

## Revendications

1. Microorganisme contenant une déficience pour la recombinaison (recA-) et une déficience en ce qui concerne la décomposition du rhamnose (rhaB-) qui est survenue en raison d'une délétion du gène rhaB.

2. Microorganisme selon la revendication 1, **caractérisé en ce qu'**il s'agit d'E. coli.

3. Microorganisme selon les revendications 1 et 2, déposé sous le numéro DSM 14459.

4. Microorganisme selon l'une quelconque des revendications 1 à 3, contenant au moins un système d'expression inductible par rhamnose.

5. Procédé de production de microorganismes selon les revendications 1 à 4, **caractérisé en ce que** la construction de mutagenèse destinée à l'inactivation du gène de la rhamnulokinase est transférée par conjugaison dans le microorganisme actif pour la recombinaison et **en ce que**, après une intégration réussie, l'activité de recombinaison est éliminée de manière ciblée.

6. Procédé selon la revendication 5, **caractérisé en ce que** la durée de conjugaison est ajustée à ≤ 30 min.

7. Utilisation du microorganisme selon une ou plusieurs des revendications 1 à 4, dans un procédé de production de protéines rec.

8. Utilisation selon la revendication 7, **caractérisée en ce que** le microorganisme possède une protéine rec ayant la propriété d'une hydantoïnase, d'une carbamoylase et/ou d'une hydantoïne ou carbamoyle racémase.

9. Utilisation du microorganisme selon une ou plusieurs des revendications 1 à 4, pour la production de mutants ou de variants du microorganisme.
